# FASCICULE DE BREVET EUROPEEN

(11) **EP 1 506 407 B1**
(45) Date de publication et mention de la délivrance du brevet: **28.06.2006**
(21) Numéro de dépôt: 03743923.9
(22) Date de dépôt: 13.03.2003
(51) Int. Cl.: G01N 33/574

(54) **PROCEDE DE DETECTION ET DE QUANTIFICATION DE CELLULES TUMORALES CIRCULANTES ISSUES DE CANCERS SOLIDES**
VERFAHREN ZUM NACHWEIS UND ZUR QUANTIFIZIERUNG VON -AUS SOLIDEN TUMOREN ABSTAMMENDEN- ZIRKULIERENDEN TUMORZELLEN
METHOD OF DETECTION AND QUANTIFICATION OF CIRCULATING TUMOUR CELLS DERIVED FROM SOLID TUMOURS

(30) Priorité: 13.03.2002 FR 0203136
(43) Date de publication de la demande: 16.02.2005
(73) Titulaire: BIOMERIEUX, 69282 Marcy-L'Etoile (FR); Centre Hospitalier Universitaire de Montpellier, Laboratoire de Virologie de l'Hopital Lapeyronie, 34000 Montpellier (FR)
(72) Inventeur: VENDRELL, Jean-Pierre, F-34170 Catelnau-le-lez (FR); PANABIERES, Catherine, F-34830 Clapiers (FR); CHOQUET-KASTYLEVSKY, Geneviève, F-69340 Francheville (FR); JOLIVET, Michel, F-69720 Saint Bonnet de Mure (FR)
(74) Mandataire: Guerre, Dominique
(86) Numéro de dépôt international: PCT/FR2003/000806
(87) Numéro de publication internationale: WO 2003/076942

(56) Documents cités:
- WO-A-99/41613
- US-A- 6 107 049
- US-B1- 6 316 213
- CORDOBA FRANCK ET AL: "Spontaneous monoclonal immunoglobulin-secreting peripheral blood mononuclear cells as a marker of disease severity in multiple myeloma." BRITISH JOURNAL OF HAEMATOLOGY, vol. 108, no. 3, mars 2000 (2000-03), pages 549-558, XP002233211 ISSN: 0007-1048
- EKERFELT C ET AL: "Detection of spontaneous and antigen-induced human interleukin-4 responses in vitro: comparison of ELISPOT, a novel ELISA and real-time RT-PCR" JOURNAL OF IMMUNOLOGICAL METHODS, ELSEVIER SCIENCE PUBLISHERS B.V.,AMSTERDAM, NL, vol. 260, no. 1-2, 1 février 2002 (2002-02-01), pages 55-67, XP004332560 ISSN: 0022-1759
- GHOSSEIN R A ET AL: "Molecular detection of micrometastases and circulating tumor cells in solid tumors" CLINICAL CANCER RESEARCH, THE AMERICAN ASSOCIATION FOR CANCER RESEARCH, US, vol. 5, no. 8, août 1999 (1999-08), pages 1950-1960, XP002225510 ISSN: 1078-0432 cité dans la demande

## Description

La présente invention concerne le domaine du diagnostic biologique en cancérologie. Plus particulièrement, la présente invention a pour objet un procédé de détection et/ou quantification de cellules tumorales circulantes capables de relarguer ou sécréter *in vitro* un ou plusieurs marqueurs tumoraux, ainsi que l'utilisation de ce procédé dans le diagnostic précoce et le pronostic de la pathologie, dans le choix des traitements thérapeutiques et l'évaluation de leur efficacité, ainsi que dans le diagnostic des rechutes dans le cadre de cancers solides.

Le diagnostic actuel de cancers consiste en un diagnostic clinique, tel que palpation mammaire dans le cas du cancer du sein, et/ou un examen paraclinique, tel que mammographie, scanner, la confirmation étant effectuée par une analyse histologique telle que biopsie ou intervention chirurgicale.

Le diagnostic clinique ou paraclinique précoce du cancer est difficile, notamment du fait du manque d'accessibilité anatomique des zones cancéreuses. En conséquence, de nombreuses tumeurs ne sont généralement mises en évidence que tardivement.

Le même problème se rencontre lorsque les zones sont anatomiquement accessibles. Par exemple, dans le cas du cancer du sein, lorsque qu'une tumeur est détectée au cours d'une mammographie, elle a souvent une évolution infraclinique de 8 ans en moyenne.

A l'heure actuelle, il n'existe pas ou peu de méthode diagnostique biologique permettant à elles seules le diagnostic de cancer.

Les méthodes de diagnostic biologique actuellement développées permettent de suivre l'évolution d'un cancer déjà diagnostiqué ou de dépister une récidive, par exemple par dosage de certains marqueurs tumoraux. Ainsi sont dosés des marqueurs sériques ou urinaires par des techniques bien connues de l'homme du métier.

En revanche, la détection directe de cellules tumorales circulantes est peu explorée et il n'existe pas de test de routine.

La possibilité de détecter des cellules tumorales circulantes a été étudiée principalement avec deux méthodes de diagnostic qui sont la cytométrie en flux et la réaction de polymérisation en chaîne (PCR) couplée à la rétrotranscriptase-PCR (RT-PCR) (Racila E., Euhus D., Weiss A., Rao C., McConnell J., Terstappen L., Uhr J. Detection and characterization of carcinoma cells in the blood. *Proc. Natl. Acad. Sci. USA* 95:4589-4594 (1998), Ghossein R., Bhattacharya S., Rosai J. Molecular détection of micrometastase and circulating tumor cells in solid tumors. *Clin. Cancer* 5,1950-1960 (1999) et Moss, T.J., 1991, *N. Engl. J. Med.,* 324, 219-226). Ces deux méthodes ont permis de détecter, chez des patients atteints de cancer du sein et de la prostate, des cellules circulantes tumorales dans des cellules du sang ou de la moelle osseuse.

Toutefois, ces deux méthodes présentent des inconvénients. En particulier, aucune de ces méthodes ne permet de quantifier des cellules rares circulantes issues d'une tumeur solide. De plus, il est possible d'obtenir des faux positifs avec la PCR de sorte que cette technique manque de sensibilité et de spécificité. En conséquence, on a habituellement besoin de tissu tumoral pour confirmer la présence de cellules tumorales.

Racila, E. et al. (1998, *supra*) ont décrit une méthode de détection et caractérisation de cellules de cancers du sein et de la prostate dans le sang, combinant un enrichissement immunomagnétique de cellules épithéliales avec une analyse en cytométrie en flux, puis, en cas de réponse positive, une analyse immunocytochimique. L'analyse immunocytochimique est basée sur la détection colorimétrique d'une enzyme couplée à un marqueur tumoral (anticorps anti-cytolcératine 5, 6, 8 et 18). Ce test nécessite un anticorps primaire spécifique du marqueur de cancer, une immunoglobuline secondaire de lapin, une immunoglobuline de souris anti-phosphatase alcaline, la phosphatase alcaline et le substrat correspondant.

La méthode ci-dessus présente les inconvénients suivants :
- elle nécessite un appareillage spécifique coûteux, notamment pour l'analyse en cytométrie en flux,
- deux analyses sont nécessaires, à savoir une analyse en cytométrie en flux suivie d'une analyse immunocytochimique et
- elle manque de sensibilité.

De plus, aucune des méthodes précédemment citées ne permet de s'assurer de la viabilité des cellules circulantes et de leur potentiel de survie.

Cordoba F., et al. (2000, British Journal of Haemalology, 108, 549-558) ont décrit un procédé de détection de cellules myélumateuses de patients atteints d'un myélome multiple en utilisant la propriété connue de ces cellules à sécréter de l'immunoglobuline. Les cellules myélomateuses sont issues de lymphocytes B normalement présents dans le sang et normalement sécréteurs d'immunoglobulines.

Zhou Zegi et al ont décrit dans le brevet US 6 107 049 un kn de diagnostic, comprenant une surface de culture préalablement revêtue d'un ou plusieurs partenaires de liaison, des marqueurs tumoraux spécifiques du cancer prostatique.

Ces marqueurs, anticorps anti-PSA, permettent une mesure sélective de l'antigène (CPSA) spécifique de la prostate, et une haute sensibilité de ce test de diagnostic qui permet la limitation du recours à des biopsies.

La Demanderesse a maintenant trouve de manière surprenante que les cellules tumorales circulantes issues de cancers solides étaient capables de relarguer ou sécréter certains marqueurs tumoraux et qu'il était possible de détecter cette sécrétion.

La demanderesse a ainsi mis au point un nouveau procédé de détection et/ou quantification de cellules tumorales circulantes issues de cancers solides utilisant cette caractéristique particulière de relargage ou sécrétion de marqueurs tumoraux et palliant les inconvénients ci-dessus, à savoir qu'il est simple à mettre en oeuvre en ce sens qu'il ne comporte qu'une seule étape d'analyse et qu'il ne nécessite pas de matériel spécifique. De plus, ce procédé permet de détecter des cellules circulantes rares du fait de sa sensibilité très élevée et permet également de s'assurer de la viabilité desdites cellules tumorales. Il est donc très utile tant en diagnostic qu'en exploration de la maladie résiduelle et qu'en évaluation du potentiel de survie, donc d'agressivité, de ces cellules circulantes.

Ainsi, la présente invention a pour objet un procédé de détection et/ou quantification de cellules tumorales circulantes d'un échantillon biologique, lesquelles sont capables de relarguer ou sécréter *in vitro* un au plusieurs marqueurs tumoraux, comprenant les étapes consistant à :
(i) déposer une quantité connue desdites cellules au fond d'une surface de culture sur laquelle ust fixé au moins un partenaire de liaison spécifique dudit ou desdits marqueurs tumoraux,
(ii) cultiver lesdites cellules en conditions telles qu'elles relarguent ou sécrètent lesdits marqueurs tumoraux qui sont immunocapturés au fond de la surface de culture,
(iii) éliminer les cellules par lavage,
(iv) ajouter au moins un conjugué marqué spécifique desdits marqueurs tumoraux et
(v) visualiser le marquage ainsi obtenu.

Le procédé de l'invention permet donc de dénombrer des cellules néoplasiques non hématopoïétiques circulantes provenant d'échantillons biologiques de patients atteints d'un cancer solide.

Les cancers solides sont bien connus de l'homme du métier. A titre d'exemple, on peut citer les cancers du sein, de la prostate, de la thyroïde, du foie, des testicules, des ovaires, du système digestif, du poumon, etc.

Les échantillons biologiques susceptibles de contenir des cellules tumorales circulantes comprennent tout fluide biologique tel que le sang, la moelle osseuse, les épanchements, le lait, le liquide céphalo-rachidien et les urines.

Selon un mode de réalisation préféré, les échantillons biologiques sont constitués par le sang ou la moelle osseuse.

Les marqueurs tumoraux sont des marqueurs spécifiques des cancers solides qui sont capables d'être relargués ou sécrétés par des cellules tumorales *in vivo* ou *in vitro* sous certaines conditions de culture.

On entend par marqueur relargué d'une cellule tumorale un marqueur membranaire qui a été clivé et on entend par marqueur sécrété par une cellule tumorale tant un marqueur sécrété directement par ladite cellule, qu'un marqueur qui a été clivé dans le cytoplasme, puis excrété par ladite cellule tumorale.

A titre de marqueur, on peut citer différents antigènes (protéiques ou non protéiques).

Selon un mode de réalisation préféré, lesdits marqueurs tumoraux sont soit des antigènes membranaires capables d'être relargués par clivage au fond de la surface de culture, soit des antigènes intracellulaires qui sont sécrétés par lesdites cellules au fond de la surface de culture.

A titre d'exemple d'antigène membranaire, on peut citer la protéine Muc-1 qui est une protéine de surface des cellules du cancer du sein et qui est clivée sous forme de protéine CA15-3 (carbohydrate 15-3).

A titre d'exemple d'antigène sécrété, on peut citer le PSA qui est produit par les cellules du cancer de la prostate, la protéine cathépsine-D (Cath-D) qui est une aspartyl protéase du lysosome exprimée dans tous les tissus mais qui est surexprimée par les cellules cancéreuses dans le cadre du cancer du sein, la thyroglobuline (TG) produite par les cellules cancéreuses dans le cadre du cancer de la thyroïde, la protéine CA 125 produite par les cellules cancéreuses dans le cadre du cancer de l'ovaire, les protéines ACE et CA 19-9 produite par les cellules cancéreuses dans le cadre du cancer colorectal et l'alpha foetoprotéine (AFP) produite par les cellules hépatiques dans le cadre des hépatocarcinomes.

Selon un mode de réalisation préféré de l'invention, les cellules tumorales sont capables de relarguer comme marqueur tumoral la protéine CA15-3 et le cancer recherché est le cancer du sein.

Selon un autre mode de réalisation préféré de l'invention, les cellules tumorales sont capables de sécréter comme marqueur tumoral la protéine TG et le cancer recherché est le cancer de la thyroïde.

Selon un autre mode de réalisation préféré de l'invention, les cellules tumorales sont capables de re-sécréter comme marqueur tumoral la protéine CA 125 et le cancer recherché est le cancer de l'ovaire.

Selon un autre mode de réalisation préféré de l'invention, les cellules tumorales sont capables de sécréter comme marqueur tumoral les protéines ACE et CA 19-9 et le cancer recherché est le cancer colorectal.

Selon un autre mode de réalisation préféré de l'invention, les cellules tumorales sont capables de sécréter comme marqueur tumoral l'alpha foetoprotéine et le cancer recherché est le cancer primaire du foie.

Selon un autre mode de réalisation préféré de l'invention, les cellules tumorales sont capables de sécréter comme marqueur tumoral le PSA et le cancer recherché est le cancer de la prostate.

Les partenaires de liaison spécifiques des marqueurs tumoraux sont constitués par tout partenaire susceptible de se lier avec les marqueurs tumoraux. A titre d'exemple, on peut citer les anticorps, les fractions d'anticorps et les protéines.

Les anticorps partenaires de liaison sont soit des anticorps polyclonaux, soit des anticorps monoclonaux.

Les anticorps polyclonaux peuvent être obtenus par immunisation d'un animal avec au moins un antigène tumoral d'intérêt, suivie de la récupération des anticorps reclaerclaés sous forme purifiée, par prélèvement du sérum dudit animal, et séparation desdits anticorps des autres constituants du sérum, notamment par chromatographie d'affinité sur une colonne sur laquelle est fixée un antigène spécifiquement reconnu par les anticorps, notamment un antigène tumoral d'intérêt.

Les anticorps monoclonaux peuvent être obtenus par la technique des hybridomes dont le principe général est rappelé ci-après.

Dans un premier temps, on immunise un animal, généralement une souris, (ou des cellules en culture dans le cadre d'immunisations *in vitro*) avec un antigène tumoral d'intérêt, dont les lymphocytes B sont alors capables de produire des anticorps contre ledit antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de l'antigène tumoral d'intérêt pourront être testées par exemple en ELISA, par immunotransfert en une ou deux dimensions, en immunofluorescence, ou à l'aide d'un biocapteur. Les anticorps monoclonaux ainsi sélectiomés, sont par la suite purifiés notamment selon la technique de chromatographie d'affinité décrite ci-dessus.

Des exemples de fractions d'anticorps partenaires de liaison des marqueurs tumoraux comprennent les anticorps anti-CA15-3, anti-PSA, anti-alpha foeto-protéine, anti-thyroglobuline, anti-CA 19-9 et anti-CA 125.

Dans le cas du cancer du sein où les cellules sont capables de relarguer la protéine CA15-3, on pourra utiliser, comme partenaire de liaison, des anticorps anti-CA15-3.

Dans le cas du cancer de la prostate où les cellules sont capables de sécréter le PSA, on pourra utiliser, comme partenaire de liaison, des anticorps anti-PSA.

Dans le cas du cancer de la thyroïde où les cellules sont capables de sécréter de la TG, on pourra utiliser, comme partenaire de liaison, des anticorps anti-TG.

Dans le cas du cancer de l'ovaire où les cellules sont capables de sécréter du CA 125, on pourra utiliser, comme partenaire de liaison, des anticorps anti-CA 125.

Dans le cas du cancer colorectal où les cellules sont capables de sécréter du CA 19-9 ou de l'ACE, on pourra utiliser, comme partenaire de liaison, des anticorps anti-CA 19-9 et anti-ACE.

Dans le cas de l'hépatocarcinome où les cellules sont capables de sécréter de l'alpha foetoprotéine, on pourra utiliser, comme partenaire de liaison, des anticorps anti-AFP.

La surface de culture peut contenir plusieurs partenaires de liaison. De préférence, la surface de culture contient jusqu'à quatre partenaires de liaison différents.

Selon un mode de réalisation préféré, la surface de culture contient deux types d'anticorps différents dirigés contre des antigènes spécifiques du cancer du sein, de préférence les anticorps anti-CA15-3 et anti-Cath-D.

La surface de culture est telle qu'elle permet la culture de cellules hunorales. A titre d'exemple, on peut citer les micropuits, les microplaques, les surfaces plastiques et les membranes.

Le micropuits ou la microplaque eux-mêmes peuvent être constitués de plastique de sorte que les partenaires de liaison sont fixés directement au micropuits ou à la microplaque. Ils peuvent également contenir une membrane classiquement connue de l'homme du métier, laquelle est susceptible de fixer les partenaires de l'invention. A titre d'exemple, on peut citer les membranes en nitrocellulose et en Immobilon-P (Millipore Corporation).

L'échantillon biologique de patients d'intérêt est déposé directement au fond de la surface de culture ou bien les cellules non hémapoïétiques sont enrichies avant dépôt sur ledit fond.

Dans le cas des prélèvements sanguins, les cellules sont enrichies par exemple grâce à une technique de séparation cellulaire sur Ficoll associée à une déplétion des cellules sanguines utilisant des anticorps anti-CD45 couplés à des billes magnétiques (Dynal Biotech ASA, Norvège). Dans ces conditions, quelques cellules circulantes tumorales par millilitre de sang total peuvent être dénombrées.

Toute autre méthode d'enrichissement connue de l'homme du métier convient aux fins de l'invention.

Le dénombrement des cellules déposées sur la membrane d'un micropuits est effectué par hemacytométrie (cellule de Thomas, Kovas slide ).

Les conditions de culture permettant le relarguage ou la sécrétion des marqueurs tumoraux sont des conditions classiques telles que 37°C sous atmosphère hiunide et à 5% de CO₂.

L'élimination des cellules après immunocapture des marqueurs tumoraux par les partenaires de liaison fixés au fond de la surface de culture est effectuée par lavages consistant à utiliser des tampons de lavage classiques tels que le tampon PBS (Phosphate Buffered Saline) avec ou sans albumine bovine (1%).

Les conjugués, utilisés après élimination des cellules, sont des conjugués classiquement connus de l'homme du métier.

A titre d'exemple de conjugué, on peut citer les anticorps monoclonaux et les anticorps polyclonaux. De préférence, les anticorps conjugués ont une spécificité épitopique différente des anticorps fixés au fond de la surface de culture.

Par marquage des conjugués, on entend la fixation d'un marqueur capable de générer directement ou indirectement un signal détectable. Une liste non limitative de ces marqueurs consiste en :
- les enzymes qui produisent un signal détectable par exemple par colorimétrie, fluorescence, luminescence, comme la péroxydase de raifort, la phosphatase alcaline, la α-galactosidase, la glucose-6-phosphate déshydrogénase,
- les chromophores comme les composés fluorescents, luminescents, colorants,
- les molécules radioactives comme le ³²P, le ³⁵S ou le ¹²⁵I, et
- les molécules fluorescentes telles que l'alexa ou les phycocyanines.

Des systèmes indirects peuvent être aussi utilisés, comme par exemple des ligands capables de réagir avec un anti-ligand. Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/complémentaire du polynucléotide. Dans ce cas, c'est le ligand qui porte l'agent de liaison. L'anti-ligand peut être détectable directement par les marqueurs décrits au paragraphe précédent ou être lui-même détectable par un ligand/anti-ligand.

Ces systèmes de détection indirects peuvent conduire, dans certaines conditions, à une amplification du signal. Cette technique d'amplification du signal est bien connue de l'homme du métier, et l'on pourra se reporter aux demandes de brevet antérieures FR98/10084 ou WO-A-95/08000 de la demanderesse ou à l'article J. Histochem. Cytochem. 45 : 481-491, 1997.

Selon le type de marquage du conjugué utilisé, l'homme du métier ajoutera des réactifs pennettant la visualisation du marquage.

Ainsi, par exemple, dans le cas des enzymes, il est nécessaire d'ajouter un substrat chromogène, tel que le NBT-BCPI pour la phosphatase alcaline ou l'AEC pour la peroxydase. L'addition du substrat chromogène laisse alors apparaître à l'endroit où se trouvait une cellule cible un précipité coloré ou immuno-spot (bleu avec le NBT-BCIP et rouge avec l'AEC), véritable empreinte protéique laissée par la cellule.

L'ensemble des immuno-spots présents au fond de la surface de culture peut être visualisé et dénombré à la loupe binoculaire ou mieux à l'aide de lappareillage KS ELISPOT (Société Carl Zeiss Vision GmbH) équipé d'un microscope performant et d'une caméra numérique couplés à un système informatique.

Pour le marquage en fluorescence, l'ensemble des immunospots est visualisé et dénombré avec l'appareillage KS ELISPOT adapté à l'étude de la fluorescence.

Les critères retenus pour l'analyse de ces spots incluent le diamètre, la couleur, la forme, la saturation, le contraste et le gradient de diffusion. En effet, la densité et la granulosité des spots décroît du centre vers la périphérie suivant un gradient de diffusion très caractéristique d'une synthèse protéique.

Dans le cas où plus de deux partenaires de liaison sont présents dans la surface de culture, la révélation du couplage entre partenaire de liaison-marqueur tumoral est effectuée de préférence avec des anticorps secondaires marqués avec des fluorochromes.

Ainsi, selon un mode de réalisation préféré, les étapes (iv) à (v) de l'invention sont remplacées par les étapes suivantes :
(iv') ajouter des anticorps secondaires marqués avec des fluorochromes,
(v') visualiser la fluorescence lorsqu'il y a couplage entre partenaire de liaison et marqueur tumoral.

Le dénombrement des cellules tumorales par le procédé de l'invention permet de mesurer leur capacité de migration dans les cancers solides. Le pronostic, le suivi de l'efficacité des traitements thérapeutiques administrés, la quantification de la maladie résiduelle et le diagnostic des rechutes infracliniques et biologiques dans les cancers solides sont donc rendus possibles grâce au procédé de l'invention du fait de sa très grande sensibilité et spécificité.

De plus, les tumeurs peuvent relarguer des cellules tumorales circulantes dès le début de leur formation de sorte que le procédé de l'invention permet un diagnostic précoce de cancer.

En conséquence, un autre objet de l'invention consiste en l'utilisation du procédé de l'invention dans le diagnostic précoce et le pronostic de la pathologie, dans le choix et l'évaluation de l'efficacité des traitements thérapeutiques et dans le diagnostic des rechutes dans le cadre de cancers solides.

Selon un mode de réalisation préféré, le procédé de l'invention est utilisé dans le diagnostic du cancer du sein, de la prostate, de la thyroïde, de l'ovaire, du colon, du rectum et du foie.

Par ailleurs, le procédé de l'invention couple une méthode de détection spécifique avec une méthode de culture et permet donc de s'assurer du caractère viable et fonctionnel des cellules tumorales détectées, cette propriété étant importante dans le cadre du pronostic.

Un autre objet de l'invention consiste donc en l'utilisation du procédé de l'invention pour évaluer le potentiel de survie des cellules tumorales circulantes issues de patients atteints de cancers solides.

En effet, un résultat positif du procédé de l'invention met en évidence un tel potentiel de survie.

Le procédé de l'invention peut être mis en oeuvre grâce à un kit de diagnostic comprenant une surface de culture préalablement revêtue d'un ou plusieurs partenaires de liaison des marqueurs tumoraux spécifiques du cancer dont on veut effectuer la recherche, le ou les conjugués correspondants préalablement marqués. Le kit peut également contenir les solutions pour le lavage drastique des cellules après i mmunocapture.

Bien entendu, le procédé de l'invention peut être utilisé pour dénombrer toute cellule tumorale circulante capable de relarguer ou sécréter au moins un marqueur identifié comme marqueur tumoral pour lequel il existe un partenaire spécifique de liaison.

Ainsi, par exemple, le procédé de l'invention permet de dénombrer :
- des cellules thyroïdiennes tumorales productrices de thyroglobuline (TG) ou de calcitonine (CT),
- des cellules hépatiques tumorales productrices d'alpha-foetoprotéines (AFP),
- des cellules testiculaires tumorales productrices d'AFP ou d'hormone choronic gonadotropine (béta-HCG),
- des cellules mammaires tumorales productrices de CA15-3, de cathépsine D, de PS2, d' Her2/neu, de mammaglobine B,
- des cellules ovariennes tumorales productrices de CA-125,
- des cellules prostatiques tumorales productrices de PSA,
- des cellules tumorales du système digestif (colon, rectum, estomac et pancréas) productrices de CA19-9, CA-125, de CA 19-9 et d'ACE, et
- des cellules tumorales du mélanome productrices de la protéine S 100.

La présente invention sera mieux comprise à l'aide des exemples suivants donnés uniquement à titre illustratif et non limitatif, ainsi qu'à l'aide des figures 1 à 3 annexées, sur lesquelles :
- la figure 1 (1A à 1F) montre les immunp-spots obtenus selon le procédé de l'invention à partir de cellules tumorales MCF-7,
- la figure 2 montre les immuno-spots obtenus selon le procédé de l'invention à partir de cellules CD45(-) de sujets témoin et de sujets atteints du cancer du sein métastasique,
- la figure 3 montre les immuno-spots obtenus selon le procédé de l'invention chez un sujet parmi ceux atteints du cancer du sein métastasique.

### Exemple 1 : Dénombrement des cellules tumorales provenant des lignées cellulaires tumorales MCF-7 et MDA-MB-231 (cancer du sein)

On a utilisé la lignée MCF-7 car elle secrète des niveaux élevés des protéines Cath-D et MUC1, ainsi que la lignée MDA-MB-231 car elle n'exprime que la protéine Cath-D.

Les lignées cellulaires MCF-7 et MDA-MB-231 ont été maintenues dans un milieu de Eagle modifié de Dulbecco (DMEM, biochrom KG, Berlin, Allemagne) complémenté avec 1% de glutamax (Lifes technologies, Paisley, Ecosse), 10% de sérum de veau foetal (Life Technologies), 500 UI/ml de pénicilline et 500µg/ml de streptomycine (Life Technologies) dans un incubateur humidifié contenant 5% de CO₂ à 37°C.

Des plaques de microtitrage de 96 puits (Nunc, Roskilde, Danemark) utilisant une membrane de Immobilon-P comme phase solide (Millipore Corporation, Bedford, MA, U.S.A) ont été revêtues d'anticorps monoclonaux anti-cathepsine D D7E3 (Garcia, M., Capony, F., Derocq, D., Simon, D., Pau, B., & Rochefort, H. Characterization of monoclonal antibodies to the estrogen-regulated Mr 52,000 glycoprotein and their use in MCF7 cells. *Cancer Research* 45, 709-716 (1985) et d'anticorps monoclonaux anti-CA15-3 (Dakocytomation, France), et ont été laissées toute la nuit à +4°C. Les anticorps non liés à la membrane ont été éliminés en lavant trois fois avec du PBS. Les sites non liés ont ensuite été bloqués avec de la sérum albumine bovine à 5% (Sigma-Aldrich, St Quentin Fallavier, France) pendant une heure à la température ambiante.

Si nécessaire, les lignées cellulaires ont été traitées avec 50µg/ml de cycloheximide sur un dispositif permettant à la fois l'inclinaison et la rotation à 37°C pendant une heure, avant d'effectuer les dosages.

Les cellules viables provenant des lignées cellulaires ont été comptées dans un hématocytomètre après coloration par exclusion au colorant bleu trypan, puis diluées en série dans les puits en double dans un milieu de croissance à différentes concentrations. Les plaques ont ensuite été incubées à 37°C dans du CO₂ à 5% pendant 24 heures.

Après lavage avec du PBS, on a ajouté soit des anticorps monoclonaux anti-Cath-D M1G8 (Garcia, M., Capony, F., Derocq, D., Simon, D., Pau, B., & Rochefort, *supra*) conjugués à la peroxydase de raifort, soit des anticorps monoclonaux anti-CA15-3 DF3 (Dakocytomation) conjugués à la phosphatase alcaline (procédé une couleur), soit un mélange de ceux-ci (procédé à deux couleurs) et les plaques ont été incubées à la température ambiante.

Le substrat chromatique approprié, à savoir kit de coloration AEC (Sigma-aldrich) pour la peroxydase de raifort et mélange de sel de X-phosphate/5-bromo-4-cliloro-3-indolyl-phosphate toluidine et de chlorure de 4-Nitro blue tétrazolium (BCIP/NTB, Sigma), a été ajouté dans chaque puits. On a obtenu des précipités insolubles de couleur rouge (peroxydase/présence de Cath-D) ou bleue (phosphatase alcaline/présence de CA15-3) en 5 à 10 minutes.

Les plaques ont ensuite été lavées avec de l'eau distillée pour stopper la réaction.

Les immuno-spots ont été comptés en utilisant l'appareillage KS ELISPOT. Les puits sans cellules ou sans revêtement d'anticorps spécifiques ont été inclus en tant que témoin.

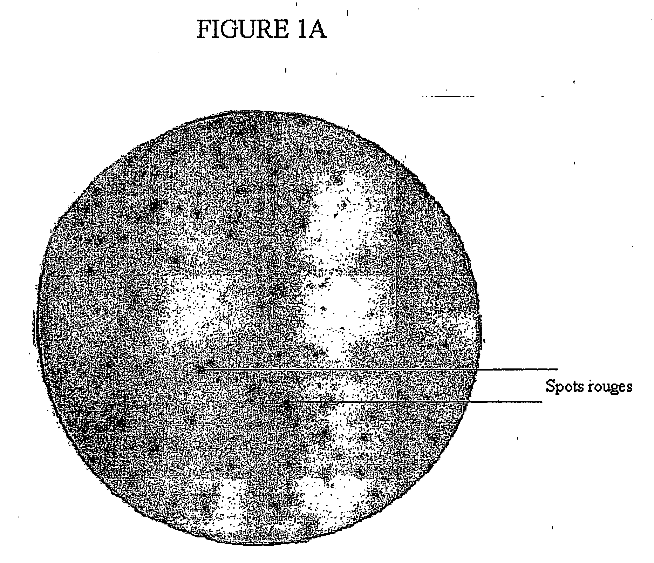

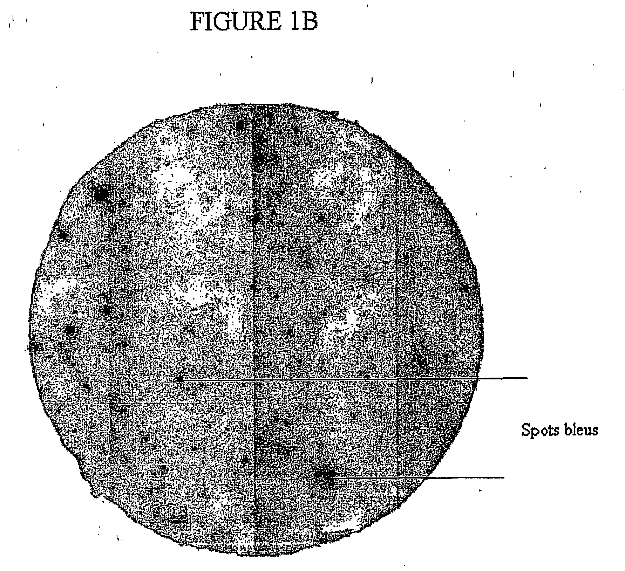

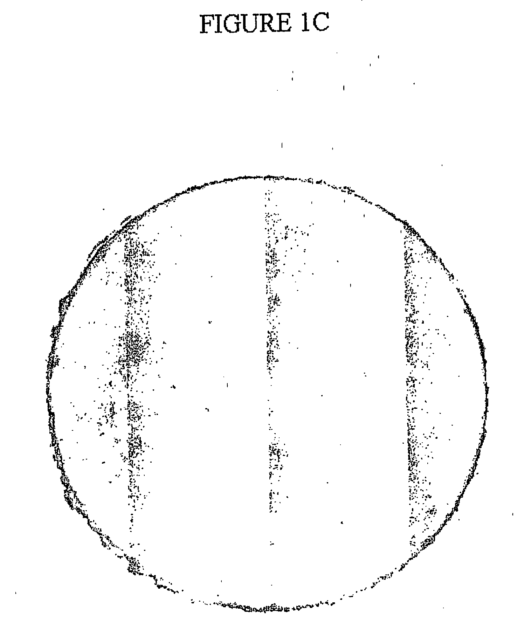

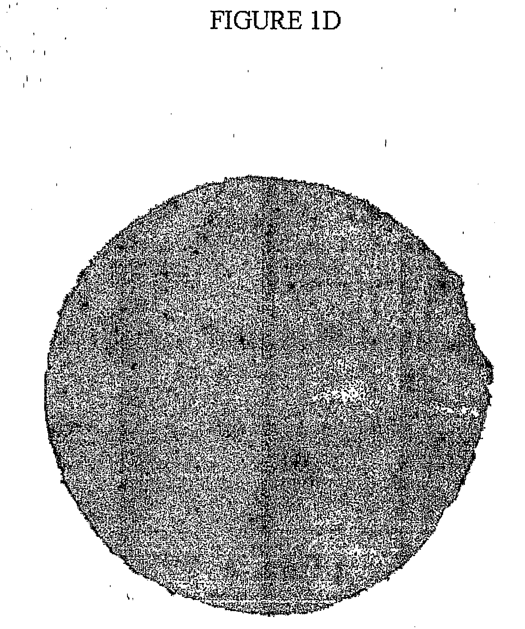

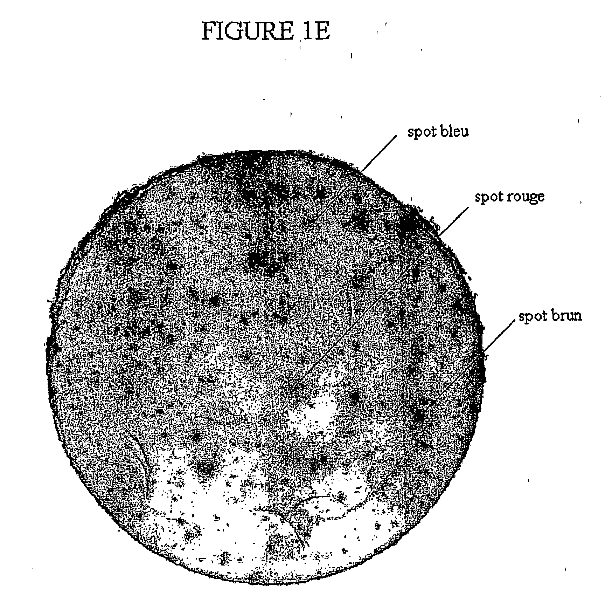

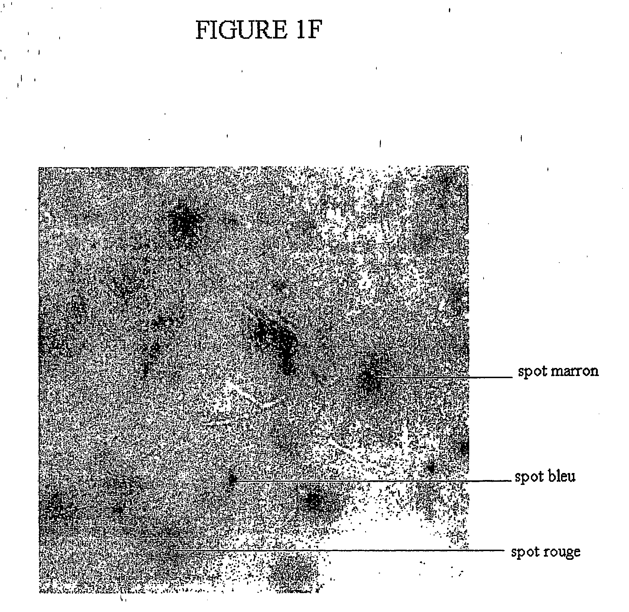

La figure 1 montre les résultats obtenus. Comme montré sur la figure 1 A-B (1A pour Cath-D ; 1B pour CA15-3) où le procédé une couleur a été utilisé, l'utilisation d'une combinaison d'anticorps monoclonaux anti-Cath-D et anti-CA15-3 permet d'observer qu'environ 25% des cellules MCF-7 sécrètent les protéines Cath-D et/ou CA15-3 après 24 heures de culture *in vitro.* L'addition de cycloheximide pendant la culture diminue à la fois la taille et le nombre de spots obtenus, ce qui confirme une synthèse protéique *de novo* (figure 1 C-D pour Cath-D-CA15-3, respectivement).

Il est à noter que les cellules de la lignée MDA-MB-231 n'ont montré des spots qu'avec les anticorps anti-Cath-D (données non-représentées).

Enfin, la technique à deux couleurs (figure 1 E-F) permet d'observer qu'avec les lignées cellulaires MCF-7, environ 17% des spots ne sont que des spots « Cath-D » (précipité de couleur rouge), 82% ne sont que des spots « CA15-3 (MUC-1) » (précipité de couleur bleue) et 1% sont des doubles spots « Cath-D » et « CA15-3 (MUC-1) » (précipité de couleur brune).

### Exemple 2 : Sensibilité de détection du procédé de l'invention

Pour déterminer le niveau de détection minimal du procédé de l'invention, on a utilisé des dilutions en série des cellules de la lignée cellulaire MCF-7, de 100 000, 10 000, 1 000, 100, 10 à 1 cellules par puits, et on a mesuré la sécrétion de Cath-D par le procédé de l'invention selon le mode opératoire décrit dans l'exemple 1 et par la technique ELISA (CisBioInternational, Saclay, France) dans le surnageant de culture correspondant.

Les résultats sont indiqués dans le tableau 1 ci-dessous :

**Tableau 1 Comparaison de la sensibilité du procédé de l'invention et de ELISA**

| cellules MCF-7 | Cath-D Procédé de | Cath-D ELISA |
|---|---|---|
| | l'invention | |
| (cellules/par puits) | (cellules/par puits) | (pmol/ml de surnageant) |
| 100.000 | ≈20.000 | 2.7 |
| 10.000 | 2350 | 0.2 |
| 1.000 | 246 | 0 |
| 100 | 22 | 0 |
| 10 | 2 | 0 |
| 1 | 0.25* | 0 |

| | | |
|---|---|---|
| *: moyenne de 4 puits. | | |

Comme montré dans le tableau ci-dessus, avec une dilution de 100 000 à 10 000 de cellules par puits, les spots étaient si nombreux qu'on ne pouvait pas les compter, tandis que la détection de Cath-D était possible avec la technique ELISA. Avec une dilution de 1000 cellules par puits, on a compté environ 250 spots correspondant à des cellules sécrétant Cath-D (25%) avec le procédé de l'invention, tandis qu'avec la technique ELISA, aucune sécrétion de Cath-D n'a été détectée. On observe des résultats identiques avec des dilutions supérieures.

Il convient de noter que même avec une seule cellule par puits, on détecte un spot avec le procédé de l'invention et qu'on a obtenu des résultats similaires avec la protéine MUC1.

Ces données montrent donc que le procédé de l'invention a une sensibilité 10 000 fois supérieure à celle de la technique ELISA appliquée à la détection de marqueur tumoral dans le surnageant de culture.

### Exemple 3 : Détection de cellules circulantes

On a isolé des cellules épithéliales circulantes et des cellules mononucléées périphériques par centrifugation par gradient de densité Ficoll-Hypaque (Pharmacia, Uppsala, Suède), de 8-10 ml d'échantillons sanguins provenant de 16 patientes présentant un cancer du sein métastatique traitées au Centre de Recherche et de Lutte contre le Cancer, Val d'Aurelle, Montpellier, France.

On a testé l'efficacité de l'isolement des cellules épithéliales de la façon suivante : on a dilué dans du sang de sujets normaux des cellules de la lignée cellulaire MCF-7 à différentes concentrations (1000, 100 et 10 cellules par ml de sang). On a ensuite traité ces quantités aliquotées de sang par gradient de Ficoll-Hypaque et on a éliminé les cellules d'origine hématologique par tri magnétique en utilisant des billes recouvertes d'anticorps monoclonaux anti-CD45. On a recherché les protéines CA15-3 dans les cellules restantes avec le procédé de l'invention. L'efficacité du dénombrement par le procédé de l'invention a été de 67%, ce qui met en évidence que le procédé de l'invention permet la récupération des cellules circulantes rares d'origine tumorale.

On a enrichi les cellules non hématopoïétiques par déplétion de toutes les cellules sanguines CD45(+) de lignée hématopoïétique provenant des cellules mononucléées périphériques en utilisant des anticorps anti-CD45 avec un marquage magnétique et un procédé de séparation magnétique selon les recommandations de Dynal Biotech ASA.

On a mis en oeuvre le procédé de l'invention sur les cellules ainsi enrichies de patientes atteintes du cancer du sein métastatique et de patients témoins selon le mode opératoire décrit dans l'exemple 1.

Les résultats sont donnés dans le tableau 2 ci-dessous :

**Tableau 2 Comptage de spots Catli-D et/ou MUC1 provenant de cellules CD45(-)**

| | | | spots / 10 ml de sang | | |
|---|---|---|---|---|---|
| Groupes | Sujets | cellules CD45(-) | | | |
| | | (x10⁶)/10 ml de sang | Cath-D | MUC-1* | Cath-D+MLTC-1 |
| ST | 1 | 5 | 0 | ND | ND |
| | 2 | 1,3 | 0 | ND | ND |
| | 3 | 0,01 | ND | 0 | ND |
| | 4 | 0,01 | ND | 0 | ND |
| | 5 | 0,01 | ND | 0 | ND |
| | 6 | 0,01 | ND | 0 | ND |
| | 7 | 0,01 | ND | 0 | ND |
| | 8 | 0,01 | ND | 0 | ND |
| | 9 | 5,4 | 0 | 0 | 0 |
| | 10 | 0,2 | 0 | 0 | 0 |
| | 11 | 0,7 | 0 | 0 | 0 |
| CSM | 12 | 0,9 | 2 | 5 | 0 |
| | 13 | 1,2 | 7 | 15 | 0 |
| | 14 | 5 | 255 | 345 | 0 |
| | 15 | 2 | 12 | 250 | 0 |
| | 16 | 0,7 | 4 | 38 | 0 |
| | 17 | 1,4 | 68 | 96 | 0 |
| | 18 | 1 | 35 | 18 | 0 |
| | 19 | 1,8 | 0 | 204 | 0 |
| | 20 | 3,9 | 217 | 48 | 0 |
| | 21 | 0,9 | 16 | 39 | 0 |
| | 22 | 1,1 | 18 | 107 | 20 |
| | 23 | 0,8 | 9 | 1700 | 10 |
| | 24 | 0,6 | 20 | 25 | 0 |
| | 25 | 1,1 | 182 | 13 | 0 |
| | 26 | 1,2 | 6 | 19 | 0 |
| | 27 | 2,3 | 2 | 48 | 0 |
| ST: sujets témoin, CSM : cancer du sein métastasique | | | | | |
| * seuls les spots >1200µ² ont été comptés. | | | | | |

Comme indiqué dans le tableau ci-dessus, lorsque les sujets témoins ont été testés, aucune expression de la protéine Cath-D n'a été détectée pour 5/5 des sujets contrôle testés.

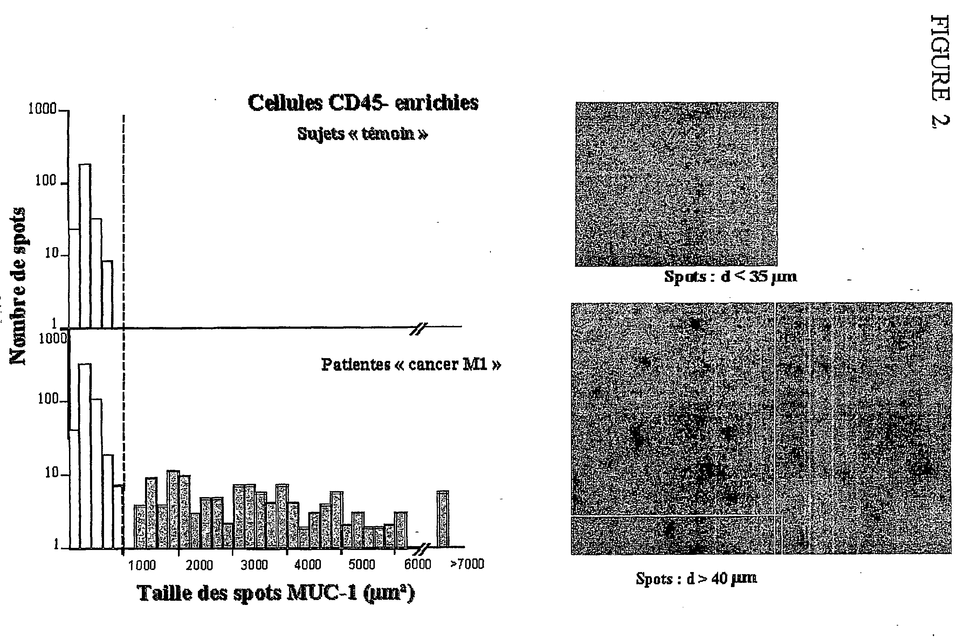

Lorsqu'on a regardé les spots CA15-3 (MUC-1) chez 9 patientes du même groupe, on a noté de nombreux spots dont la taille est inférieure à 1 000 µm² pour de nombreux sujets (voir la figure 2, partie supérieure). S'agissant des spots CA15-3 (MUC-1) chez les patientes atteintes de cancer, deux populations de spots ont été détectables, de petits spots dont la taille est inférieure à 1 000 µm² et des spots plus gros, d'une taille comprise entre 1 200 µm² et plus de 7 000 µm² (figure 2, partie inférieure), ce qui suggère qu'un seuil critique de 1 000 µm² pourrait être utile pour définir une expression anormale de MUC1 via CA15-3 (figure 2, partie inférieure).

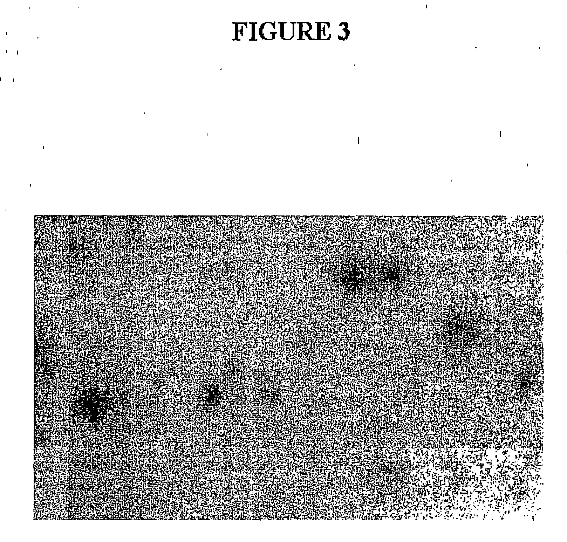

Pour les 16 patientes atteintes d'un cancer avancé, une majorité de cellules n'ont exprimé qu'un seul antigène tumoral, de 0,2 à 25,5 cellules par ml de sang pour la protéine Cath-D et de 0,5 à 170 cellules de sang pour la protéine CA15-3, tandis que seules 2 patientes parmi ces 16 ont exprimé les deux protéines (figure 3).

Pour une patiente explorée lors du premier diagnostic (c'est-à-dire sans traitement) d'une tumeur (taille < 2 cm), et avec un ganglion sentinelle envahi, nous avons dénombré 5,1 spots/ml de sang avant l'intervention chirurgicale et 0 spot/ml quatre jours après

### Exemple 4 : Dénombrement des cellules tumorales provenant des lignées cellulaires tumorales LNCAP et MCF-7 (cancer de la prostate)

On a utilisé la lignée LNCAP car elle secrète des niveaux élevés de la protéine PSA, ainsi que la lignée MCF-7 car elle n'exprime pas la protéine PSA.

La lignée cellulaire LNCAP a été maintenue dans un milieu RPMI (Eurobio, Les Ullis, France) complémenté QSP 450 ml avec 5 ml de glutamine (2 mM), 50 ml de sérum de veau foetal (10%), 100 UI/ml de pénicilline, 100 µg/ml de streptomycine, 2,5 ml de glucose 100 (4,5 g/l) et 5 ml de pyruvate de sodium 100 mM (1mM).

La lignée cellulaire MCF-7, telle que décrite dans l'exemple 1, a été utilisée comme témoin.

Des plaques de microtitrage de 96 puits (Nunc, Roskilde, Danemark) utilisant une membrane de Immobilon-P comme phase solide (Millipore Corporation, Bedford, MA, U.S.A) ont été revêtues d'anticorps monoclonaux anti-PSA (bioMérieux, Marcy l'Etoile, France) et ont été laissées toute la nuit à +4°C. Les anticorps non liés à la membrane ont été éliminés en lavant trois fois avec du PBS. Les sites non liés ont ensuite été bloqués avec de la sérumalbumine bovine à 5% (Sigma-Aldrich, St Quentin Fallavier, France) pendant une heure à la température ambiante.

Si nécessaire, les lignées cellulaires ont été traitées avec 50µg/ml de cycloheximide sur un dispositif permettant à la fois l'inclinaison et la rotation à 37°C pendant une heure, avant d'effectuer les dosages.

Les cellules viables provenant des lignées cellulaires ont été comptées' dans un hématocytomètre après coloration par exclusion au colorant bleu trypan, puis diluées en série dans les puits en double dans un milieu de croissance à différentes concentrations. Les plaques ont ensuite été incubées à 37°C dans du CO₂ à 5% pendant 24 heures.

Après lavage avec du PBS, on a ajouté des anticorps monoclonaux anti-PSA (bioMérieux) conjugués à la phosphatase alcaline (procédé une couleur), et les plaques ont été incubées à la température ambiante.

Le substrat chromatique approprié, à le mélange de sel de X-phosphate/5-bromo-4-chloro-3-indolyl-phosphate toluidine et de clilorure de 4-Nitro blue tétrazolium (BCIP/NTB, Sigma), a été ajouté dans chaque puits. On a obtenu des précipités insolubles de couleur bleue en 5 à 10 minutes.

Les plaques ont ensuite été lavées avec de l'eau distillée pour stopper la réaction.

Les immuno-spots ont été comptés en utilisant l'appareillage KS ELISPOT. Les puits sans cellules ou sans revêtement d'anticorps spécifiques ont été inclus en tant que témoin.

Il est à noter que l'efficacité de l'isolement des cellules épithéliales a été testée comme indiqué dans l'exemple 3, lequel a été de 70%.

### Exemple 5 : Détection de cellules circulantes sécrétrices de PSA :

On a isolé des cellules épithéliales circulantes et des cellules mononucléées périphériques par centrifugation par gradient de Ficoll-Hypaque (Pharmacia, Uppsala, Suède), de 8-10 ml d'échantillons sanguins provenant de 10 patients présentant un cancer de la prostate métastatique traités à la Clinique « Beau Soleil » et au CHU de Montpellier, France.

On a enrichi les cellules non hématopoïétiques par déplétion de toutes les cellules sanguines CD45(+) de lignée hématopoïétique provenant des cellules mononucléées périphériques en utilisant des anticorps anti-CD45 avec un marquage magnétique et un procédé de séparation magnétique selon les recommandations de Dynal Biotech ASA.

On a mis en oeuvre le procédé de l'invention sur les cellules ainsi enrichies de patients atteints du cancer de la prostate métastatique et de patients témoins selon le mode opératoire décrit dans l'exemple 1.

Lorsque les sujets témoins ont été testés, aucune expression de la protéine PSA n'a été détectée pour 6/6 des sujets testés. Par contre, pour 10 patients ayant des métastases osseuses, nous avons dénombré des cellules circulantes sécrétrices de PSA.

### Exemple 6 : Dénombrement des cellules tumorales provenant des lignées cellulaires tumorales ML-1 (cancer de la thyroïde)

On a utilisé la lignée ML-1 car elle sécrète des niveaux élevés de la protéine TG.

La lignée cellulaire ML-1 nous a été fournie gracieusement par l'équipe allemande de D. Grimm (Schonberger J, Bauer J, Spruss T, Weber G, Chahoud I, Billes C, Grimm D. Establishment and characterization of the follicular thyroid carcinoma cell line ML-1, and characterization of the follicular thyroid carcinoma cell line ML-1. *J Mol Med* 2000;78(2):102-10).

Elle a été maintenue dans un milieu DMEM (4,5 g/l glucose) complémenté de 20% de sérum de veau foetal, de la glutamine (2 mM) et du pyruvate de sodium (1 mM).

Des plaques de microtitrage de 96 puits (Nunc, Roskilde, Danemark) utilisant une membrane de Immobilon-P comme phase solide (Millipore Corporation, Bedford, MA, USA) ont été revêtues d'anticorps monoclonaux anti-TG (BioRad, Marnes la Coquette, France) et ont été laissées toute la nuit à +4°C. Les anticorps non liés à la membrane ont été éliminés en lavant trois fois avec du PBS. Les sites non liés ont ensuite été bloqués avec de la sérumalbumine bovine à 5% (Sigma-Aldrich, ST Quentin Fallavier, France) pendant une heure à température ambiante.

Les cellules ML-1 ont été comptées dans un hématocytomètre après coloration par exclusion au colorant bleu trypan, puis diluées en série dans les puits en double dans un milieu de croissance adéquat à différentes concentrations. Les plaques ont ensuite été incubées à 37°C dans du CO₂ à 5% pendant 24 heures.

Après lavages avec du PBS, on a ajouté des anticorps monoclonaux anti-TG (BioRad, Marnes la Coquette, France) conjugués à la phosphatase alcaline (procédé une couleur), et les plaques ont été incubées à température ambiante.

Le substrat chromatique approprié (mélange de sel de X-phosphate/5-bromo-4-chloro-3-indoyl-phosphate toluidine et de chlorure de 4-Nitro blue tétrazolium (BCIP/NTB, Sigma) a été ajouté dans chaque puits. On a obtenu des précipités insolubles de couleur bleue en 5 à 10 minutes.

Les plaques ont ensuite été lavées avec de l'eau distillée pour stopper la réaction.

Les immuno-spots ont été comptés en utilisant l'appareillage KS ELISPOT. Les puits sans cellules ou sans revêtement d'anticorps spécifiques ont été inclus en tant que témoin.

Il est à noter que l'efficacité de l'isolement des cellules épithéliales a été testée comme indiqué dans l'exemple 3, lequel a été de 70%.

### Exemple 7 : Détection de cellules circulantes sécrétrices de TG:

On a isolé des cellules épithéliales circulantes et des cellules mononucléées périphériques par centrifugation par gradient de Ficoll-Hypaque (Pharmacia, Uppsala, Suède), de 8-10 ml d'échantillons sanguins provenant de 15 patients présentant un cancer de la thyroïde métastatique traités à l'hôpital Lapeyronie au CHU de Montpellier, France.

On a enrichi les cellules non hématopoïétiques par déplétion de toutes les cellules sanguines CD45(+) de lignée hématopoïétique provenant des cellules mononucléées périphériques en utilisant des anticorps anti-CD45 avec un marquage magnétique et un procédé de séparation magnétique selon les recommandations de Dynal Biotech ASA.

On a mis en oeuvre le procédé de l'invention sur les cellules ainsi enrichies de patients atteints du cancer de la thyroïde métastatique et de patients témoins selon le mode opératoire décrit dans l'exemple 1.

Nous avons inclus dans notre étude uniquement des patient(e)s ayant des taux sériques de TG positif. On a exploré 15 patient(e)s à distance du traitement de la tumeur primaire. Pour 5/6 patient(e)s en évolution métastatique, nous avons dénombré des cellules circulantes sécrétrices de TG ; pour 3 patient(e)s, ce nombre de cellules était augmenté après stimulation *in vivo* par thyrogène. Pour deux patientes, des spots ont été dénombrés sans complications cliniques et avec des taux normaux de TG.

### Exemple 8 : Dénombrement des cellules tumorales provenant des lignées cellulaires tumorales BG-1 et SKOV3 (cancer de l'ovaire)

On a utilisé la lignée BG-1 car elle sécrète des niveaux élevés de la protéine CA 125 ainsi que la lignée SKOV3 car elle n'exprime pas la protéine CA 125.

La lignée cellulaire BG-1 a été maintenue dans du milieu McCoy's 5a contenant de la glutamine (1,5 mM) et du sérum de veau foetal (10%).

La lignée cellulaire SKOV3 a été utilisée comme témoin. Elle a été maintenue dans un milieu identique à celui cité pour la lignée BG-1.

Des plaques de microtitrage de 96 puits (Nunc, Roskilde, Danemark) utilisant une membrane de Immobilon-P comme phase solide (Millipore Corporation, Bedford, MA, USA) ont été revêtues d'anticorps monoclonaux anti-CA125 (Dakocytomation) et ont été laissées toute la nuit à +4°C. Les anticorps non liés à la membrane ont été éliminés en lavant trois fois avec du PBS. Les sites non liés ont ensuite été bloqués avec de la sérumalbumine bovine à 5% (Sigma-Aldrich, ST Quentin Fallavier, France) pendant une heure à température ambiante.

Les cellules BG-1 ont été comptées dans un hématocytomètre après coloration par exclusion au colorant bleu trypan, puis diluées en série dans les puits en double dans un milieu de croissance adéquat à différentes concentrations. Les plaques ont ensuite été incubées à 37°C dans du CO2 à 5% pendant 24 heures.

Après lavages avec du PBS, on a ajouté des anticorps monoclonaux anti-CA125 (Dakocytomation) conjugués à la phosphatase alcaline (procédé une couleur), et les plaques ont été incubées à température ambiante.

Le substrat chromatique approprié (mélange de sel de X-phosphate/5-bromo-4-chloro-3-indoyl-phosphate toluidine et de chlorure de 4-Nitro-blue tétrazolium (BCIP/NTB, Sigma)) a été ajouté dans chaque puits. On a obtenu des précipités insolubles de couleur bleue en 5 à 10 minutes.

Les plaques ont ensuite été lavées avec de l'eau distillée pour stopper la réaction.

Les immuno-spots ont été comptés en utilisant l'appareillage KS ELISPOT. Les puits sans cellules ou sans revêtement d'anticorps spécifiques ont été inclus en tant que témoin.

Il est à noter que l'efficacité de l'isolement des cellules épithéliales a été testée comme indiqué dans l'exemple 3, lequel a été de 70%.

### Exemple 9 : Dénombrement des cellules tumorales provenant des lignées cellulaires tumorales Caco2 et HT-29 (cancer colorectal)

On a utilisé les lignées Caco2 et HT-29 car elles sécrètent des niveaux élevés des protéines CA 19-9 et ACE. Les deux lignées cellulaires ont été testées pour les deux marqueurs tumoraux.

La lignée cellulaire Caco2 a été maintenue dans un milieu MEM avec des sels de Earles et des acides aminés non essentiels, additionné de sérum de veau foetal (20%), de glutainine (2mM), de pyruvate de sodium (1 mM) et de bicarbonate de sodium (1,5 g/l).

La lignée cellulaire HT-29 a été maintenue dans un milieu du milieu McCoy's 5a contenant de la glutamine (1,5 mM) et du sérum de veau foetal (10%).

Des plaques de microtitrage de 96 puits (Nunc, Roskilde, Danemark) utilisant une membrane de Immobilon-P comme phase solide (Millipore Corporation, Bedford, MA, USA) ont été revêtues d'anticorps monoclonaux anti-CA 19-9 (Dalcocytomation) ou anti-ACE (bioMérieux, Marcy l'Etoile, France) et ont été laissées toute la nuit à +4°C. Les anticorps non liés à la membrane ont été éliminés en lavant trois fois avec du PBS. Les sites non liés ont ensuite été bloqués avec de la sérumalbumine bovine à 5% (Sigma-Aldrich, ST Quentin Fallavier, France) pendant une heure à température ambiante.

Les cellules Caco-2 et HT-29 ont été comptées dans un hématocytomètre après coloration par exclusion au colorant bleu trypan, puis diluées en série dans les puits en double dans un milieu de croissance adéquat à différentes concentrations. Les plaques ont ensuite été incubées à 37°C dans du CO2 à 5% pendant 24 heures.

Après lavages avec du PBS, on a ajouté des anticorps monoclonaux anti-CA 19-9 conjugués à la phosphatase alcaline ou anti-ACE conjugués à la peroxydase (bioMérieux, Marcy l'Etoile, France) (procédé une couleur), et les plaques ont été incubées à température ambiante.

Le substrat chromatique approprié pour la phosphatase alcaline (mélange de sel de X-phosphate/5-bromo-4-chloro-3-indoyl-phosphate toluidine et de chlorure de 4-Nitro blue tétrazolium (BCIP/NTB, Sigma) a été ajouté dans chaque puits. On a obtenu des précipités insolubles de couleur bleue en 5 à 10 minutes. Le substrat chromatique approprié pour la peroxydase, à savoir le kit de coloration AEC (Sigma-aldrich), a été ajouté dans chaque puits. On a obtenu des précipités insolubles de couleur rouge en 10 minutes.

Les plaques ont ensuite été lavées avec de l'eau distillée pour stopper la réaction.

Les immuno-spots ont été comptés en utilisant l'appareillage KS ELISPOT. Les puits sans cellules ou sans revêtement d'anticorps spécifiques ont été inclus en tant que témoin.

Il est à noter que l'efficacité de l'isolement des cellules épithéliales a été testée comme indiqué dans l'exemple 3, lequel a été de 70%.

### Exemple 10 : Dénombrement des cellules tumorales provenant des lignées cellulaires tumorales hépatiques (cancer primaire du foie)

On a utilisé la lignée hépatique car elle sécrète des niveaux élevés de l'alphaprotéine.

La lignée cellulaire a été maintenue dans un milieu a été maintenue dans du milieu RPMI contenant de la glutamine (1,5 mM) et du sérum de veau foetal (20%).

Des plaques de microtitrage de 96 puits (Nunc, Roskilde, Danemark) utilisant une membrane de Immobilon-P comme phase solide (Millipore Corporation, Bedford, MA, USA) ont été revêtues d'anticorps monoclonaux anti-CA125 (Dakocytomation) et ont été laissées toute la nuit à +4° C. Les anticorps non-lies à la membrane ont été éliminés en lavant trois fois avec du PBS. Les sites non liés ont ensuite été bloqués avec de la sérum-albumine bovine à 5% (Sigma-Aldrich, ST Quentin Fallavier, France) pendant une heure à température ambiante.

Les cellules ont été comptées dans un hématocytomètre après coloration par exclusion au colorant bleu trypan, puis diluées en série dans les puits en double dans un milieu de croissance adéquat à différentes concentrations. Les plaques ont ensuite été incubées à 37°C dans du CO2 à 5% pendant 24 heures.

Après lavages avec du PBS, on a ajouté des anticorps monoclonaux anti-AFP (bioMérieux, Marcy l'Etoile, France) conjugués à la peroxydase (procédé une couleur), et les plaques ont été incubées à température ambiante.

Le substrat chromatique approprié pour la peroxydase, à savoir le kit de coloration AEC (Sigma-Aldrich), a été ajouté dans chaque puits. On a obtenu des précipités insolubles de couleur rouge en 10 minutes.

Lés plaques ont ensuite été lavées avec de l'eau distillée pour stopper la réaction.

Les immuno-spots ont été comptés en utilisant l'appareillage KS ELISPOT. Les puits sans cellules ou sans revêtement d'anticorps spécifiques ont été inclus en tant que témoin.

Il est à noter que l'efficacité de l'isolement des cellules épithéliales a été testée comme indiqué dans l'exemple 3, lequel a été de 70%.

## Revendications

1. Procédé de détection et/ou quantification de cellules tumorales néoplasiques non hématopoïétiques circulantes d'un échantillon biologique d'un patient atteint de cancer solide, lesquelles sont capables de relarguer ou sécréter *in vitro* un ou plusieurs marqueurs tumoraux, comprenant les étapes consistant à :
(i) déposer ledit échantillon, dont on a dénombré les cellules, au fond d'une surface de culture sur laquelle est fixé au moins un partenaire de liaison spécifique dudit ou desdits marqueurs tumoraux,
(ii) cultiver lesdites cellules en conditions telles que lesdites cellules tumorales néoplasiques non hématopoïétiques relarguent ou sécrètent lesdits marqueurs tumoraux qui sont immunocapturés au fond de la surface de culture,
(iii) éliminer les cellules par lavage,
(iv) ajouter au moins un conjugué marqué spécifique desdits marqueurs tumoraux et
(v) visualiser le marquage ainsi obtenu.

2. Procédé selon la revendication 1, **caractérisé en ce que** les échantillons biologiques sont constitués par le sang ou la moelle osseuse.

3. Procédé selon la revendication 1 ou 2, **caractérisé en ce que** lesdits marqueurs tumoraux sont soit des antigènes membranaires capables d'être relargués par clivage au fond de la surface de culture, soit des antigènes intracellulaires qui sont sécrétés par lesdites cellules au fond de la surface de culture.

4. Procédé selon l'une quelconque des revendications 1 à 3, **caractérisé en ce que** quatre partenaires de liaison différents au plus, de préférence deux partenaires au plus, sont fixés au fond de la surface de culture.

5. Procédé selon l'une quelconque des revendications 1 à 3, **caractérise en ce que** les cellules tumorales sont capables de sécréter comme marqueur tumoral l'antigène PSA, et le partenaire de liaison est un anticorps anti-PSA.

6. Procédé selon l'une quelconque des revendications 1 à 3, **caractérisé en ce que** les cellules tumorales sont capables de relarguer comme marqueur tumoral l'antigcne protéique CA15-3, et le partenaire de liaison est un anticorps anti-CA 15-3.

7. Procédé selon l'une quelconque des revendications 1 à 3, **caractérisé en ce que** les cellules tumorales sont capables de sécréter comme marqueur tumoral l'antigène protéique TG, et le partenaire de liaison est un anticorps anti-TG.

8. Procédé selon l'une quelconque des revendications 1 à 3, **caractérisé en ce que** les cellules tumorales sont capables de sécréter comme marqueur tumoral l'antigène protéique CA 125, et le partenaire de liaison est un anticorps anti-CA 125.

9. Procédé selon l'une quelconque des revendications 1 à 3, **caractérisé en ce que** les cellules tumorales sont capables de sécréter comme marqueur tumoral les antigènes protéiques ACE et/ou CA 19-9, et les partenaires de liaison sont les anticorps anti-ACE et anti-CA19-9.

10. Procédé selon l'une quelconque des revendications 1 à 3, **caractérisé en ce que** les cellules tumorales sont capables de sécréter comme marqueur tumoral l'antigène protéique alpha foetoprotéine, et le partenaire de liaison est un anticorps anti-AFP.

11. Procédé selon l'une quelconque des revendications 1 à 4, **caractérisé en ce que** les partenaires de liaison sont au nombre de deux et sont de préférence des anticorps anti-CA15-3 et anti-Cath-D.

12. Procédé selon la revendication 11, **caractérisé en ce que** les étapes (iv) à (v) sont remplacées par les suivantes :
(iv') ajouter des anticorps secondaires marqués avec des fluorochromes,
(v') visualiser la fluorescence lorsqu'il y a couplage entre partenaire de liaison et marqueur tumoral.

13. Utilisation ex vivo du procédé selon l'une quelconque des revendications 1 à 12 dans le diagnostic précoce et le pronostic de la pathologie, dans le choix et l'évaluation de l'efficacité des traitements thérapeutiques et dans le diagnostic des rechutes dans le cadre de cancers solides.

14. Utilisation selon la revendication 13, **caractérisée en ce que** le cancer est le cancer du sein.

15. Utilisation selon la revendication 13, **caractérisée en ce que** le cancer est le cancer de la prostate.

16. Utilisation selon la revendication 13, **caractérisée en ce que** le cancer est le cancer de la thyroïde.

17. Utilisation selon la revendication 13, **caractérisée en ce que** le cancer est le cancer de l'ovaire.

18. Utilisation selon la revendication 13, **caractérisée en ce que** le cancer est le cancer colorectal.

19. Utilisation selon la revendication 13, **caractérisée en ce que** le cancer est le cancer primaire du foie.

20. Utilisation du procédé selon l'une quelconque des revendications 1 à 12 pour évaluer le potentiel de survie des cellules tumorales circulantes issues de patients atteints de cancers solides.

21. Utilisation d'un kit de diagnostic comprenant une surface de culture préalablement revêtue d'un ou plusieurs partenaires de liason des marqueurs tumoraux spécifiques du cancer solide dont on veut effectuer la recherche, le ou les conjugués correspondants préalablement marqués, pour détecter et/ou quantifier les cellules tumorales néoplastiques non hématopoïtiques circulantes d'un échantillon biologique d'un patient atteint dudit cancer solide.

## Claims

1. Method of detection and/or quantification of circulating nonhaematopoietic neoplastic tumour cells of a biological sample from a patient suffering from a solid cancer, which are capable of releasing or secreting *in vitro* one or more tumour markers, comprising the steps consisting in:
(i) depositing said sample, the cells of which have been counted, at the bottom of a culture surface to which at least one binding partner specific for said tumour marker (s) is attached,
(ii) culturing said cells under conditions such that said nonhaematopoietic neoplastic tumour cells release or secrete said tumour markers, which are immunocaptured at the bottom of the culture surface,
(iii) removing the cells by washing,
(iv) adding at least one labelled conjugate specific for said tumour markers, and
(v) visualizing the labelling thus obtained.

2. Method according to Claim 1, **characterised in that** the biologica7, samples consist of blood or bcne marrow.

3. Method according to Claim 1 or 2, **characterised in that** said tumour markers are either membrane antigens capable of being released by cleavage at the bottom of the culture surface, or intracellular antigens which are secreted by said cells at the bottom of the culture surface.

4. Method according to any one of Claims 1 to 3, **characterized in that** at most four different binding partners, preferably at most two partners, are attached at the bottom of the culture surface.

5. Method according to any one of Claims 1 to 3, **characterized in that** the tumour cells are capable of secreting the PSA antigen as tumour marker, and the binding partner is an anti-PSA antibody.

6. Method according to any one of Claims 1 to 3, **characterized in that** the tumour cells are capable of releasing the CA15-3 protein antigen as tumour marker, and the binding partner is an anti-CA15-3 antibody.

7. Method according to any one of Claims 1 to 3, **characterized in that** the tumour cells are capable of secreting the TG protein antigen as tumour marker, and the binding partner is an anti-TG antibody.

8. Method according to any one of Claims 1 to 3, **characterized in that** the tumour cells are capable of secreting the CA 125 protein antigen as tumour marker, and the binding partner is an anti-CA 125 antibody.

9. Method according to any one of Claims 1 to 3, **characterized in that** the tumour cells are capable of secreting the ACE and/or CA 19-9 protein antigens as tumour marker, and the binding partners are anti-ACE and anti-CA19-9 antibodies.

10. Method according to any one of Claims 1 to 3, **characterized in that** the tumour cells are capable of secreting the alpha-foetoprotein protein antigen as tumour marker, and the binding partner is an anti-AFP antibody.

11. Method according to any one of Claims 1 to 4, **characterized in that** the number of binding partners is two, and they are preferably anti-CA15-3 and anti-Cath-D antibodies.

12. Method according to Claim 11, **characterized in that** steps (iv) and (v) are replaced with the following steps:
(iv') adding secondary antibodies labelled with fluorochromes,
(v') visualizing the fluorescence when there is coupling between binding partner and tumour marker.

13. Use, ex vivo, of the method according to any one of Claims 1 to 12, in the early diagnosis and the prognosis of the pathology, in the choice and the evaluation of the effectiveness of the therapeutic treatments and in the diagnosis of relapses in the case of solid cancers.

14. Use according to Claim 13, **characterized in that** the cancer is breast cancer.

15. Use according to Claim 13, **characterized in that** the cancer is prostate cancer.

16. Use according to Claim 13, **characterized in that** the cancer is thyroid cancer.

17. Use according to Claim 13, **characterized in that** the cancer is ovarian cancer.

18. Use according to Claim 13, **characterized in that** the cancer is colorectal cancer.

19. Use according to Claim 13, **characterized in that** the cancer is primary liver cancer.

20. Use of the method according to any one of Claims 1 to 12, for evaluating the survival potential of circulating tumour cells derived from patients suffering from solid cancers.

21. Use of a diagnostic kit comprising a culture surface precoated with one or more binding partners of the tumour markers specific for the solid cancer for which it is desired to carry out the investigation, and the corresponding prelabelled conjugate(s), for detecting and/or quantifying the circulating nonhaematopoietic neoplastic tumour cells of a biological sample from a patient suffering from said solid cancer.

## Patentansprüche

1. Verfahren zur Detektion und/oder Quantifizierung von nicht-hämatopoetischen, tumorösen, neoplastischen Zellen, die in einer biologischen Probe eines Patienten, der von einem soliden Krebs betroffen ist, zirkulieren, und die in der Lage sind, *in vitro* einen oder mehrere Tumormarker abzustoßen oder zu sekretieren, das die folgenden Schritte aufweist, nämlich:
(i) Bereitstellen der Probe, deren Zellen gezählt wurdcn, auf dem Boden einer Kultivierungsoberfläche, auf der zumindest ein spezifischer Bindungspartner für den oder die Tumormarker fixiert ist,
(ii) Kultivieren der Zellen unter solchen Bedingungen, unter denen die nicht-hämatopoetischen, tumorösen, neoplastischen Zellen die Tumormarker abstoßen oder sekretieren, die auf dem Boden der Kultivierungsoberfläche immunkomplexiert sind,
(iii) Entfernen der Zellen durch Waschen,
(iv) Hinzugeben von zumindest einem für die Tumormarker spezifischen markierten Konjugat, und
(v) Visualisieren der so erhaltenen Merkierung.

2. Verfahren nach Anspruch 1, **dadurch gekennzeichnet, dass** die biologischen Proben Blut oder Knochenmark aufweisen.

3. Verfahren nach Anspruch 1 oder 2, **dadurch gekennzeichnet, dass** die Tumormarker entweder Membranantigene sind, die in der Lage sind, durch Spaltung an dem Boden der Kultivierungsoberfläche abgestoßen zu werden, oder intrazelluläre Antigene sind, die von den Zellen an dem Boden der Kultivierungsoberfläche sekretiert werden.

4. Verfahren nach einem der Ansprüche 1 bis 3, **dadurch gekennzeichnet, dass** höchstens vier verschiedene Bindungspartner, vorzugsweise höchstens zwei Bindungspartner, an den Boden der Kultivierungsoberfläche fixiert sind.

5. Verfahren nach einem der Ansprüche 1 bis 3, **dadurch gekennzeichnet, dass** die Tumorzellen in der Lage sind, als Tumormarker das PSA-Antigen zu sekretieren, und der Bindepartner ein Anti-PSA-Antikörper ist.

6. Verfahren nach einem der Ansprüche 1 bis 3, **dadurch gekennzeichnet, dass** die Tumorzellen in der Lage sind, als Tumormarker das Proteinantigen CA15-3 abzustoßen, und der Bindepartner ein Anti-CA15-3-Antikörper ist.

7. Verfahren nach einem der Ansprüche 1 bis 3, **dadurch gekennzeichnet, dass** die Tumorzellen in der Lage sind, als Tumormarker das Proteinantigen TG zu sekretierer, und der Bindepartner ein Anti-TG-Antikörper ist.

8. Verfahren nach einem der Ansprüche 1 bis 3, **dadurch gekennzeichnet, dass** die Tumorzellen in der Lage sind, als Tumormarker das Proteinantigen CA 125 zu sekretieren, und der Bindepartner ein Anti-CA 125-Antikörper ist.

9. Verfahren nach einem der Ansprüche 1 bis 3, **dadurch gekennzeichnet, dass** die Tumorzellen in der Lege sind, als Tumormarker das Proteinantigen ACE und/oder CA 19-9 zu sekreticren, und der Bindepartner ein Anti-ACE- und Anti-CA 19-9-Antikörper ist.

10. Verfahren nach einem der Ansprüche 1 bis 3, **dadurch gekennzeichnet, dass** die Tumorzellen in der Lage sind, als Tumormarker das Proteinantigen Alpha-Fetoprotein zu sekretieren, und der Bindepartner ein Anti-AFP-Antikörper ist.

11. Verfahren nach einem der Ansprüche 1 bis 4, **dadurch gekennzeichnet, dass** zwei Bindepartner vorgesehen sind, die vorzugsweise Anti-CA15-3- und Anti-Cath-D-Antikörper sind.

12. Verfahren nach Anspruch 11, **dadurch gekennzeichnet, dass** die Schritte (iv) bis (v) durch die folgenden Schritte ersetzt sind:
(iv') Zugabe von mit Fluorochromen markierten sekundären Antikörpern,
(v') Visualisieren der Fluoreszenz, wenn eine Bindung zwischen dem Bindepartner und dem Tumormarker stattgefunden hat.

13. Verwendung des Verfahrens nach einem der Ansprüche 1 bis 12 *ex vivo* in der Frühdiagnose und pathologischen Prognose, in der Entscheidung und Evaluierung der Wirksamkeit von therapeutischen Behandlungen und in der Diagnose von Rückfällen in der Umgebung von festem Krebs.

14. Verwendung nach Anspruch 13, **dadurch gekennzeichnet, dass** der Krebs Brustkrebs ist,

15. Verwendung nach Anspruch 13, **dadurch gekennzeichnet, dass** der Krebs Prostatakrebs ist.

16. Verwendung nach Anspruch 13, **dadurch gekennzeichnet, dass** der Krebs Schilddrüsenkrebs ist.

17. Verwendung nach Anspruch 13, **dadurch gekennzeichnet, dass** der Krebs Ovarialkrebs ist.

18. Verwendung nach Anspruch 13, **dadurch gekennzeichnet, dass** der Krebs Colonkrebs ist.

19. Verwendung nach Anspruch 13, **dadurch gekennzeichnet, dass** der Krebs primärer Leberkrebs ist.

20. Verwendung des Verfahrens nach einem der Ansprüche 1 bis 12, um das Überlebenspotential von zirkulieranden Tumorzellen zu evaluieren, die aus Patienten stammen, die von solidem Krebs betroffen sind.

21. Verwendung eines diagnostischen Kits, des eine Kultivierungsoberfläche, die zuvor mit einem oder mehreren Bindepartnern für Tumormarker beschichtet wurde, die spezifisch sind für soliden Krebs, bezüglich dessen eine Untersuchung durchgeführt werden soll, sowie ein oder mehrere zuvor markierte entsprechendc Konjugate aufweist, um nicht-hämatopoetische, tumoröse, neoplastische Zellen zu detektieren und/oder zu quantifizieren, die in einer biologischen Probe eines Patienten zirkulieren, der von dem soliden Krebs betroffen ist.
